# EUROPEAN PATENT APPLICATION

(11) **EP 1 811 298 A1**
(43) Date of publication of application: **25.07.2007**
(21) Application number: 06124019.8
(22) Date of filing: 14.11.2006
(51) Int. Cl.: G01N 33/50

(54) **Method and kit for positively detecting toxic material**

(30) Priority: 19.01.2006 KR 20060005844
(71) Applicant: Samsung Electronics Co., Ltd., Suwon-si Gyeonggi-do (KR)
(72) Inventor: Han, Jung-im, Yongin-si Gyeonggi-do (KR); Choi, Soo-hyung, Hwaseong-si Gyeonggi-do (KR); Lee, Soo-suk, Suwon-si Gyeonggi-do (KR); Jung, Sung-ouk, Suwon-si Gyeonggi-do (KR); Park, Joon-shik, Yongin-si Gyeonggi-do (KR); Kim, Ki-eun, Seoul (KR)
(74) Representative: Grünecker, Kinkeldey, Stockmair & Schwanhäusser Anwaltssozietät

(57) **Abstract**

Provided are a method for positively detecting toxic materials within a sample including (i) contacting sub-mitochondria particles having competent mitochondrial enzymes derived from the inner membranes of mitochondria, and an electron donor which transmits electrons to an electron transfer system of the sub-mitochondria particles, with a sample to be tested, (ii) adjusting the pH of one or more pH indicators to a pH higher than the pH of the sample reaction of (i), (iii) adding the pH indicator(s) of (ii) to each sample reaction of (i), and (iv) identifying color changes of one or more pH indicators. A kit for performing said method is also provided.

All kinds of toxic materials can be detected as well as the presence of toxic materials and their degree of toxicity. In addition, no special equipment or apparatus is required and thus, simplification of the method is possible. When materials desired to be detected exist, the method of positively detecting toxic materials within a sample is the same as a general positive detection mechanism and thus, commercialization is also possible.

## Description

### BACKGROUND OF THE INVENTION

### 1. Field of the Invention

The present invention relates to a method and a kit for positively detecting the presence of toxic materials within a sample and the degree of toxicity thereof.

### 2. Description of the Related Art

A new type of damage caused by an increase of hazardous materials due to industrialization (for example, damage by endocrine disrupting chemicals), ecosystem damage caused by an increase of water pollution sources such as wastes and agricultural chemical components, serious problems caused by remaining agricultural chemicals in food, and heavy metal contamination in soil increasing on a daily basis, threaten human bodies and the environment.

Therefore, there is a significant and urgent need to monitor the harmfulness of chemical materials which have a bad influence on human bodies and the environment.

Various conventional biological analysis methods for detecting toxic materials within environmental samples are well known.

For example, U.S Patent No. 4,808,517 discloses an analysis method of toxic materials within an environmental sample including: mixing (a) a suspension of sub-mitochondria particles having competent mitochondrial enzymes formed from the inner membranes of mitochondria, (b) an analysis medium including a substrate in which either the substrate or an enzyme reaction product thereof can be detected using a spectroscopic detection method when the analysis medium is converted by mitochondrial enzymes, and (c) environmental samples which will be tested, in a common vessel; and determining how the sample influences enzyme activity within the suspension particles by measuring the changes of the substrate using spectroscopic measurement.

The conventional method can screen most kinds of toxic materials. However, since a spectroscopic measurement instrument is required, a simplification of the method is desirable. Also, the conventional method cannot measure a degree of toxicity of toxic materials and is a negative detection method which is different from a general positive detection mechanism and thus, commercialization is also not possible.

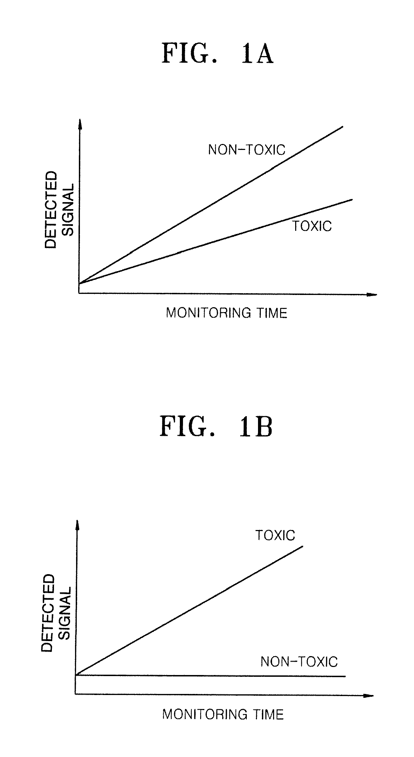

FIGS. 1A and 1 B are graphs illustrating a concept for a method of negative detection and positive detection, respectively. Referring to FIG. 1A, since negative detection measures an obstruction of a specific reaction caused by toxicity, small signal changes due to a reaction obstruction occur when toxicity exists and big signal changes due to no reaction obstruction occur when toxicity does not exist.

In contrast, referring to FIG. 1 B, in the case of positive detection, signal changes do not occur when toxicity does not exist and big signal changes occur when toxicity does exist.

Conventionally, some positive detection methods of detecting the presence of toxic materials within a sample are known. For example, a biosensor generating a signal when specific toxic materials exist using recombinant bioluminescent bacteria into which both stress promoters and lux genes are transformed (Sue Hyung Choi and Man Bock Gu. 2001: PHENOLIC TOXICITY-DETECTION AND CLASSIFICATION THROUGH THE USE OF A RECOMBINANT BIOLUMINESCENT ESCHERICHIA COLI. Environmental Toxicology and Chemistry: Vol.20, No.2, pp.248-255).

However, the conventional biosensor is selective about detecting specific toxic materials causing specific stress and thus, all the various kinds of toxic materials cannot be screened and the degree of toxicity also can not be measured.

### SUMMARY OF THE INVENTION

The present invention provides a method of positively detecting all kinds of toxic materials and their degree of toxicity.

The present invention also provides a kit for positively detecting all kinds of toxic materials and their degree of toxicity.

According to an aspect of the present invention, there is provided a method of positively detecting toxic materials within a sample comprising the steps of: (i) contacting sub-mitochondria particles having competent mitochondrial enzymes formed from the inner membranes of mitochondria and an electron donor which transmits electrons to an electron transfer system of the sub-mitochondria particles, with a sample to be tested; (ii) adjusting the pH of one or more pH indicators to a pH higher than the pH of the sample reaction of (i); (iii) adding the pH indicators of (ii) to the sample reaction of (i); and (iv) identifying color changes of one or more pH indicators, wherein a color change indicates the presence of a toxic material and non-toxicity is indicated when no color change occurs.

The sub-mitochondria particles may be manufactured by sonicating whole mitochondria to generate micelles from the mitochondrial cristae.

The mitochondrial enzymes may be selected from NADH-dehydrogenase, coenzyme-Q-cytochrome C reductase, and cytochrome C oxidase.

The electron donor may include at least one selected from the group consisting of NADH and NADPH.

The pH indicator may change color in the pH range of 5.2 to 10.

The pH indicator may be selected from the group consisting of bromocresol purple, bromothymol blue, phenol red, thymol blue, and phenolphthalein.

The pH indicator may be adjusted in the pH range of 7.5 to 11.

The pH indicator may be adjusted in the pH range of 9 to 11.

The toxicity may be indicated when the color of a pH indicator changes and non-toxicity may be indicated when the color of a pH indicator is not changed.

One or more pH indicators may be formed of more than two pH indicators and the degree of toxicity of toxic materials may be measured according to color changes of two pH indicators.

According to another aspect of the present invention, there is provided a kit for positively detecting toxic materials within a sample including: sub-mitochondria particles having competent mitochondrial enzymes derived from the inner membranes of mitochondria; an electron donor which transmits electrons to an electron transfer system of the sub-mitochondria particles; and one or more pH indicators.

The sub-mitochondria particles may be manufactured by sonicating whole mitochondria to generate micelles from the mitochondrial cristae.

The sub-mitochondria particles may be lyophilized.

The mitochondrial enzymes may be selected from NADH-dehydrogenase, coenzyme-Q-cytochrome C reductase, and cytochrome C oxidase.

The electron donor may include at least one selected from the group consisting of NADH and NADPH.

The pH indicator may change color in the pH range of 5.2 to 10.

The pH indicator may be selected from the group consisting of bromocresol purple, bromothymol blue, phenol red, thymol blue, and phenolphthalein.

The pH indicator may be adjusted in the pH range of 7.5 to 10.

The pH indicator may be adjusted in the pH range of 9 to 10.

### BRIEF DESCRIPTION OF THE DRAWINGS

The above and other features and advantages of the present invention will become more apparent by describing in detail exemplary embodiments thereof with reference to the attached drawings in which:
FIGS. 1A and 1 B are graphs illustrating a concept for a method of negative detection and positive detection, respectively;
FIG. 2 is a schematic diagram illustrating a concept for a method of positively detecting toxic materials according to an embodiment of the present invention;
FIGS. 3A and 3B are graphs illustrating the pH variables according to the time when 20 % of ethanol (toxic material) or 20 % of sorbitol (non-toxic material) are added to sub-mitochondria particles and an electron donor to cause a reaction and showing the pH of each reactant after 10 minutes of the reaction described in FIG. 3A;
FIG. 4 is a diagram schematically illustrating a method of positively detecting toxic materials in which phenol red is used as a pH indicator according to an embodiment of the present invention;
FIG. 5A is a graph illustrating the pH variables according to the time when 20 % of ethanol (toxic material), 2.5 % of ethanol (toxic material), or 20 % of sorbitol (non-toxic material) are added to sub-mitochondria particles and an electron donor to cause a reaction and FIG. 5B shows the pH of each reaction described in FIG. 5A after 10 minutes of incubation;
FIG. 6 is a diagram schematically illustrating a method of positively detecting toxic materials in which phenolphthalein is used as a pH indicator according to an embodiment of the present invention; and
FIG. 7 is a diagram schematically illustrating a method of positively detecting toxic materials and their degree of toxicity in which phenol red and phenolphthalein are used as pH indicators according to an embodiment of the present invention.

### DETAILED DESCRIPTION OF THE INVENTION

Hereinafter, the present invention will be described more fully.

A method of positive detection according to an embodiment of the present invention is to detect all kinds of toxic materials and the degree of toxicity thereof.

In an embodiment of the present invention, a method of detecting the presence of toxic materials within a sample includes a process of contacting sub-mitochondria particles having competent mitochondrial enzymes derived from the inner membranes of mitochondria, an electron donor which transmits electrons to an electron transfer system of the sub-mitochondria particles, with a sample to be tested.

The sub-mitochondria particles (SMPs) of the present embodiment are lipid bilayer membrane vesicles obtained by a micro-formation from a fraction of a crista film when mitochondria are ruptured. That is, whole mitochondria derived from arbitrary origin are ruptured by an ultrasonic wave, a digitonin or a french press process and are separated from cytoplasm residue by centrifugal separation for interception of the membrane to reform a vesicle which moves stably like the inner membrane of mitochondria. Such sub-mitochondria particles can move like whole mitochondria and a large amount of sub-mitochondria particles also can be saved by being frozen or lyophilized after manufacturing and thus, the aliquot of a sub-mitochondria particle product can be easily used in performing a toxicity assay which takes a long time.

The sub-mitochondria particles may be manufactured by sonicating whole mitochondria to generate micelles from the mitochondrial cristae.

The sub-mitochondria particles used in an embodiment of the present invention can be manufactured by any conventional method or purchased commercially. A method of manufacturing the sub-mitochondria particles is, for example, disclosed in U. S Patent No. 4,808,517. Firstly, in order to manufacture sub-mitochondria particles, whole mitochondria should be prepared. Here, mitochondria derived from an arbitrary origin may be used. The sub-mitochondria particles themselves may be manufactured from fresh mitochondria or from frozen mitochondria. Once the sub-mitochondria particles are manufactured, they can be saved in a preserved mixture at -20°C. Also, for storage purposes, the sub-mitochondria particles can be lyophilized. In order to use the lyophilized sub-mitochondria particles in an embodiment of the present invention, they can simply be defrosted. A lyophilized product can be mixed with an analysis medium before use and thus, can be simply reformed.

The sub-mitochondria particles include competent mitochondrial enzymes. The term "competent mitochondrial enzyme" of the present invention, comprises any enzymatically active enzyme involved in an electron transfer system of mitochondria. Accordingly, the method of an embodiment of the present invention refers to measuring how toxic materials affect an electron transfer system of mitochondria or an electron transfer system of sub-mitochondria particles by detecting color change. In more detail, toxic materials affect the enzymes of the electron transfer system of sub-mitochondria particles and thus, affect the amount of electrons transmitted from an electron donor to the electron transfer system. Subsequently, toxic materials affect the amount of electron donors within a reaction solution. The method of the present embodiment refers to measuring the amount of electron donors within a reaction solution by detecting color change, thereby measuring the influence on the electron transfer system, that is, the extent of toxicity. However, the present invention is not limited thereto. The principle mechanism underlying the pH change that is measured within the sample reaction is based on the enzymes of the sub-mitochondria particles. These enzymes of the electron transport chain, comprising NADH dehydrogenase, coenzyme-Q-cytochrome C reductase (i.e. the cytochrome bc1 complex) and cytochrome C oxidase, depend on electron donors such as NADH and NADPH to generate a proton gradient leading to a high concentration of protons within the inter-membrane space of the mitochondria. In the native context, these protons will be driven back into the mitochondrial matrix via an ATPase which thereby produces ATP from ADP and Pi.

When generating sub-mitochondria particles two general species of vesicles are formed, one species wherein the enzymes of the electron transport chain are orientated so as to generate a high proton concentration in their lumen and a second species wherein the enzymes are orientated inversely so. Only those vesicles which are able to acidify their lumen will be able to access and consume the electron donors supplied to the sample reaction. The pH gradient however, can not be influenced by the action of the ATPase of these vesicles due the absence of ADP in the sample reaction. Thus, the protons are "trapped" and concentrated inside the vesicles. Consequently, the pH of the sample reaction is raised. In the other, inversely orientated species of vesicles, in turn, the enzymes can not access the electron donors of the sample reaction. Thus, these vesicles will not influence the pH of the sample reaction.

When a toxic is present, the activity of the enzymes is reduced or obstructed and the resulting pH change of the sample reaction is lower or absent.

The mitochondrial enzymes may be selected from NADH-dehydrogenase, coenzyme-Q-cytochrome C reductase, and cytochrome C oxidase.

The electron donor may include at least one selected from the group consisting of NADH and NADPH.

The toxic materials may be any toxic materials which have an influence on the electron transfer system of mitochondria or sub-mitochondria particles, for example, ethanol, methanol, phenols, heavy metals, and solvents, but are not limited thereto.

The method of contacting described above can be performed in any condition as long as the enzymes of the electron transfer system can react, for example, a physiological condition or a condition similar to the physiological condition. That is, the process of contacting may be performed in a solution under physiological salt conditions and a pH near to pH 7 (for example, a PBS solution) or in a buffer for a predetermined time. When the contacting method is performed under physiological conditions or in a solution similar thereto, the concentration of the sub-mitochondria particles may be 0.1 to 0.5 mg/ml and the concentration of the electron donors may be 1 to 30 mM. However, the concentrations of sub-mitochondria particles and the electron donors are not limited to these amounts. As the concentration of the sub-mitochondria particles and the electron donors increase, the reaction speed also increases and thus, the sub-mitochondria particles and the electron donors can be used by considering the desired reaction speed and adjusting their concentration appropriately.

In the present invention, a method of detecting the presence of toxic materials within a sample includes a process of adjusting the pH of one or more pH indicators which changes color according to the pH change to a pH higher than the final pH of the sample reaction.

The pH indicator of the present invention can be any indicator as long as the color changes according to the pH change. For example, pH indicators which may be used in the present invention are illustrated in Table 1.

**<Table 1 >**

| Indicator | Color at low pH | pH range of color change | Color at high pH |
|---|---|---|---|
| Methyl violet | Yellow | 0.0~1.6 | Blue-purple |
| Thymol blue (acid, first color change) | Red | 1.2~2.8 | Yellow |
| Methyl yellow | Red | 2.9~4.0 | Yellow |
| Bromophenol blue | Yellow | 3.0~4.6 | Purple |
| Congo red | Blue | 3.0~5.2 | Red |
| Methyl orange | Red | 3.1~4.4 | Yellow |
| Litmus | Red | 4.5~8.3 | Blue |
| Bromocresol purple | Yellow | 5.2~6.8 | Purple |
| Bromothymol blue | Yellow | 6.0~7.6 | Blue |
| Phenol red | Yellow | 6.6~8.0 | Red |
| Thymol blue (base, second color change) | Yellow | 8.0~9.6 | Blue |
| Phenolphthalein | Colorless | 8.2~10.0 | Pink |
| Thymolphthalein | Colorless | 9.4~10.6 | Blue |
| Alizarin yellow R | Yellow | 10.1~12.0 | Red |
| Indigo carmine | Blue | 11.4~13.0 | Yellow |

According to an embodiment of the present invention, the pH indicators may change color in the pH range of 5.2 to 10.

According to an embodiment of the present invention, the pH indicators may be selected from the group consisting of bromocresol purple, bromothymol blue, phenol red, thymol blue, and phenolphthalein.

A pH adjustment of one or more pH indicators may be performed by a conventional method. For example, such a process may be performed by adding an acid or basic solution into the pH indicators.

According to an embodiment of the present invention, adjustment of pH indicators may be performed in the pH range of 7.5 to 11, for example, 9 to 11.

In an embodiment of the present invention, a method of detecting the presence of toxic materials within a sample includes a process of adding one or more pH indicators to each sample reaction.

The weight ratio of sample reaction to pH adjusted indicator solution is not particularly restricted and is 1:0.1 to 10, and preferably, 1:0.2 to 5, for example, 1:1.

In an embodiment of the present invention, a method of detecting the presence of toxic materials within a sample includes a process of identifying color changes of one or more pH indicators.

According to an embodiment of the present invention, when the color of the pH indicator is changed, the sample tested may be identified as being toxic and when the color is not changed, the sample tested may be identified as being non-toxic.

According to an embodiment of the present invention, one or more pH indicators may be formed of at least two pH indicators and the degree of toxicity of toxic materials may be measured according to the color change combination of these pH indicators.

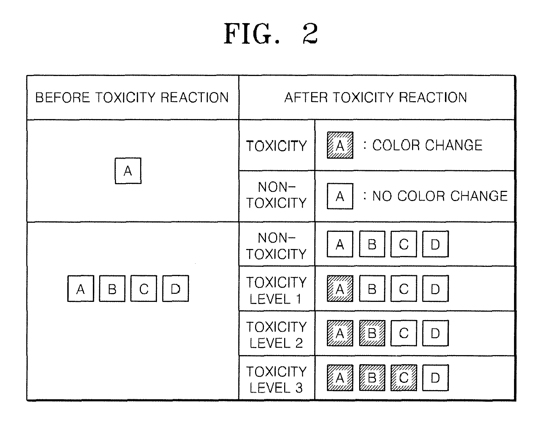

FIG. 2 is a schematic diagram illustrating a concept for a method of positively detecting toxic materials according to an embodiment of the present invention. Referring to FIG. 2, both cases of using one pH indicator and using four pH indicators are illustrated.

In the case of using one pH indicator, when toxic materials exist in the sample, the color of the pH indicators is changed upon addition to the sample after the reaction. When toxic materials do not exist in the sample, the color of the pH indicators does not change.

In the case of using four pH indicators, when no toxic materials exist in the sample, color is not changed after the reaction. When toxic materials exist in the sample, the color of a number of different pH indicators (A, B, C, D) changes according to the degree of toxicity of the toxic material.

When the degree of toxicity is low at 1, medium at 2, and high at 3, color changes occur in A, A and B, and A, B, and C among four pH indicators, respectively.

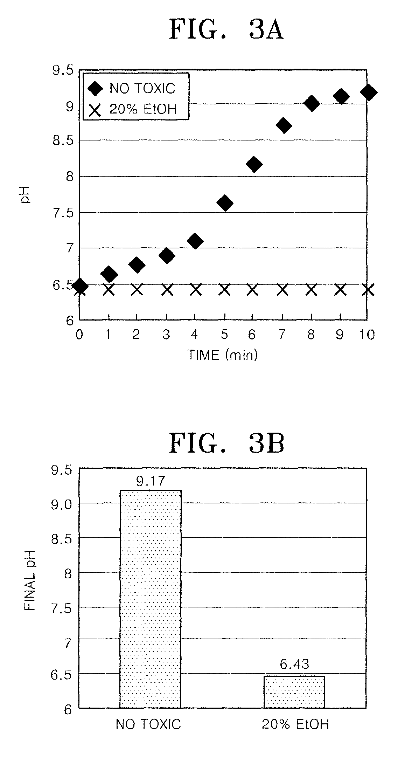

FIGS. 3A and 3B are graphs illustrating the pH variables according to the time when 20 % of ethanol (toxic material) and 20 % of sorbitol (non-toxic material) are added to sub-mitochondria particles and an electron donor to cause a reaction and showing the pH of each sample reaction after 10 minutes of the reaction described in FIG. 3A.

Referring to FIGS. 3A and 3B, when toxicity exists, the pH starts at 6.4 and 10 minutes later, the pH of the sample reaction is 6.43 showing little change. When toxicity does not exist, the pH of the sample reaction rapidly increases to 9.17.

Using the result described above, a method of positive detection using phenol red as a pH indicator according to an embodiment of the present invention is now described. That is, after phenol red is adjusted to various pH ranges, a color change is identified by adding the phenol red into the sample reaction and thus, toxic materials within a sample can be detected.

The calculation value of the above process using phenol red is illustrated in Table 2. The pH of the sample reaction having toxic materials and no toxic materials is assumed to be 6.3 and 9.3, respectively.

Referring to Table 2, when the initial pH of phenol red is adjusted to over 9, a signal (color) is the same when there are no toxic materials and the signal (color) is changed when there are toxic materials, indicating a positive mechanism.

**<Table 2>**

| Adjusted pH | Color before mixing | Mixed with 1:1 | [H⁺] | pH | Color after mixing |
|---|---|---|---|---|---|
| pH 7 | Red | pH 7+pH 6.3 (toxicity) | 3.01×10⁻⁷ | 6.52 | Red orange |
| | | pH 7+pH 9.3 (non-toxicity) | 5.03×10⁻⁸ | 7.30 | Red |
| pH 8 | Red | pH 8+pH 6.3 (toxicity) | 2.56×10⁻⁷ | 6.59 | Red orange |
| | | pH 8+pH 9.3 (non-toxicity) | 5.25×10⁻⁹ | 8.28 | Cherry |
| pH 9 | Cherry | pH 9+pH 6.3 (toxicity) | 2.51×10⁻⁷ | 6.60 | Red orange |
| | | pH 9+pH 9.3 (non-toxicity) | 7.51×10⁻¹⁰ | 9.12 | Cherry |
| pH 10 | Cherry | pH 10+pH 6.3 (toxicity) | 2.51×10⁻⁷ | 6.60 | Red orange |
| | | pH 10+pH 9.3 (non-toxicity) | 3.01×10⁻¹⁰ | 9.52 | Cherry |
| pH 11 | Cherry | pH 11+pH 6.3 (toxicity) | 2.51×10⁻⁷ | 6.60 | Red orange |
| | | pH 11+pH 9.3 (non-toxicity) | 2.56×10⁻¹⁰ | 9.59 | Cherry |

In order to verify the calculation illustrated above, actual experiments are conducted using an embodiment of the present invention.

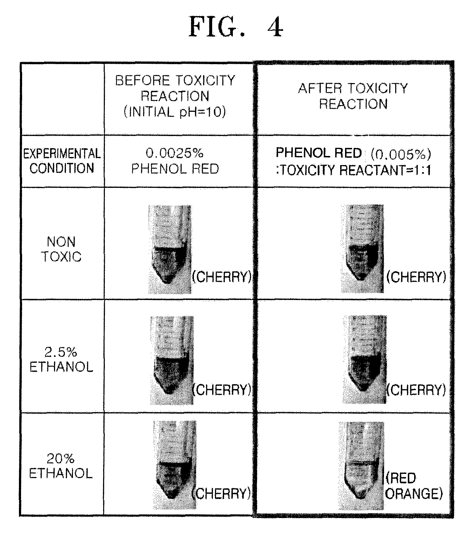

FIG. 4 is a diagram schematically illustrating a method of positively detecting toxic materials in which phenol red is used as a pH indicator. Referring to FIG. 4, after the pH of phenol red was adjusted to 10 and mixed with the sample reaction, the color was not changed both when toxic materials were not contained in the sample reaction and toxic materials of 2.5% of ethanol were contained in the sample reaction. However, when highly toxic materials of 20% of ethanol were contained in the sample reaction, the color was changed.

When phenol red is used, the presence of toxic materials can be positively detected, but as shown above, low toxicity and non-toxicity gives the same result and thus, a low degree of toxicity cannot be measured.

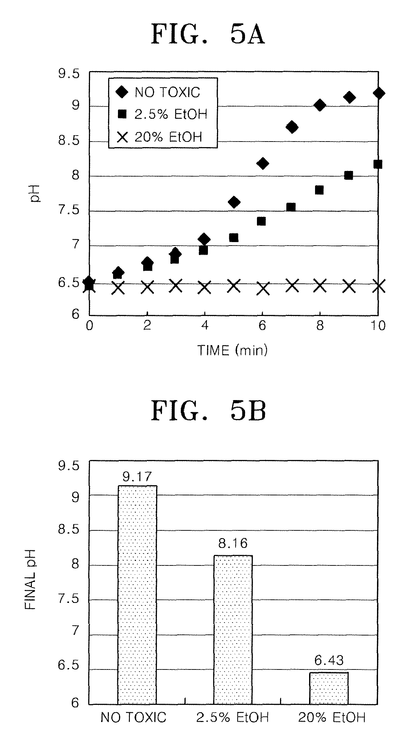

FIGS. 5A and 5B are graphs illustrating the pH variables according to the time when 20 % of ethanol (toxic material), 2.5 % of ethanol (toxic material), and 20 % of sorbitol (non-toxic material) are added to sub-mitochondria particles and an electron donor to cause a reaction and showing the pH of each sample reaction after 10 minutes of the reaction described in FIG. 5A.

Referring to FIGS. 5A and 5B, when a high degree of toxicity exists, pH starts at 6.4 and 10 minutes later, the pH of the reaction resultant is 6.43 showing little change. When a low degree of toxicity exists, the pH of the sample reaction increases to 8.16 and when non-toxicity exists, the pH of the sample reaction rapidly increases to 9.17.

Using the result described above, after phenolphthalein, which is used as a pH indicator, is adjusted to various pH ranges, a color change is identified by adding the phenolphthalein into the sample reaction and thus, toxic materials within a sample can be detected. The calculation value of the above process using phenolphthalein is illustrated in Table 3. The pH of the sample reaction having highly toxic materials, low toxic materials, and non-toxic materials are assumed to be 6.3, 8.3 and 9.3, respectively.

Referring to Table 3, when the initial pH of phenolphthalein is adjusted to over 9, a signal is the same when there are no toxic materials and the signal is changed when there are toxic materials, indicating a positive mechanism.

**<Table 3>**

| Adjusted pH | Color before mixing | Mixed with 1:1 | [H⁺] | pH | Color after mixing |
|---|---|---|---|---|---|
| pH 7 | Colorless | pH 7+pH 6.3 (high toxicity) | 3.01×10⁻⁷ | 6.52 | Colorless |
| | | pH 7+pH 8.3 (low toxicity) | 5.25×10⁻⁸ | 7.28 | Colorless |
| | | pH 7+pH 9.3 (non-toxicity) | 5.03×10⁻⁸ | 7.30 | Colorless |
| pH 8 | Colorless | pH 8+pH 6.3 (high toxicity) | 2.56×10⁻⁷ | 6.59 | Colorless |
| | | pH 8+pH 8.3 (low toxicity) | 7.51×10⁻⁹ | 8.12 | Colorless |
| | | pH 8+pH 9.3 (non-toxicity) | 5.25×10⁻⁹ | 8.28 | Colorless |
| pH 9 | Pink | pH 9+pH 6.3 (high toxicity) | 2.51×10⁻⁷ | 6.60 | Colorless |
| | | pH 9+pH 8.3 (low toxicity) | 3.01×10⁻⁹ | 8.52 | Colorless |
| | | pH 9+pH 9.3 (non-toxicity) | 7.51×10⁻¹⁰ | 9.12 | Pink |
| pH 10 | Pink | pH 10+pH 6.3 (high toxicity) | 2.51×10⁻⁷ | 6.60 | Colorless |
| | | pH 10+pH 8.3 (low toxicity) | 2.56×10⁻⁹ | 8.59 | Colorless |
| | | pH 10+pH 9.3 (non-toxicity) | 3.01×10⁻¹⁰ | 9.52 | Pink |
| pH 11 | Pink | pH 11 +pH 6.3 (high toxicity) | 2.51×10⁻⁷ | 6.60 | Colorless |
| | | pH 11 +pH 8.3 (low toxicity) | 2.51×10⁻⁹ | 8.60 | Colorless |
| | | pH 11 +pH 9.3 (non-toxicity) | 2.56×10⁻¹⁰ | 9.59 | Pink |

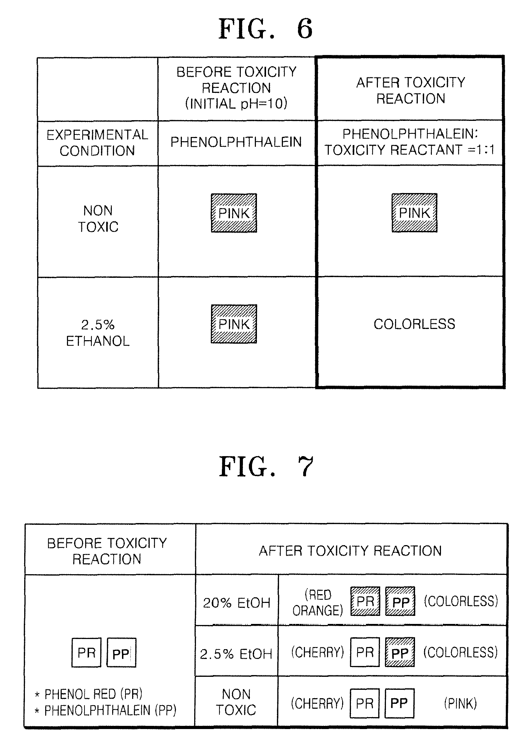

FIG. 6 is a diagram schematically illustrating a method of positively detecting toxic materials in which phenolphthalein is used as a pH indicator according to an embodiment of the present invention. Referring to FIG. 6, after the pH of phenolphthalein was adjusted to 10 and then mixed with the sample reaction, the color was not changed when toxic materials were not contained in the sample reaction and the color might be changed even when low toxic materials of 2.5 % of ethanol were contained in the sample reaction.

When phenolphthalein is used, the presence of toxic materials can be positively detected but as shown above, low toxicity and high toxicity gives the same result and thus, the degree of toxicity cannot be measured

FIG. 7 is a diagram schematically illustrating a method of positively detecting toxic materials in which phenol red and phenolphthalein are used as pH indicators according to an embodiment of the present invention.

The pH of each phenol red and phenolphthalein was adjusted to a (previously determined) pH value higher than the pH of the sample reaction and then added to the sample reaction in order to identify the color change.

Referring to FIG. 7, when toxic materials are not contained in the sample reaction, the color of both indicators was not changed. When low toxic materials of 2.5 % of ethanol were contained in the reaction resultant, the color of phenol red was not changed but color of phenolphthalein was changed. When highly toxic materials of 20 % of ethanol were contained in the reaction resultant, the color of both phenol red and phenolphthalein is changed.

As illustrated above, when more than two kinds of pH indicators are used, the presence of toxic materials can be positively detected and the degree of toxicity can also be measured.

A kit for positively detection is to detect all kinds of toxic materials and their degree of toxicity.

According to the present invention, a kit for positively detecting toxic materials includes sub-mitochondria particles having competent mitochondrial enzymes derived from the inner membranes of mitochondria, an electron donor which transmits electrons to an electron transfer system of the sub-mitochondria particles, and one or more pH indicators.

The sub-mitochondria particle may be manufactured by sonicating whole mitochondria to generate micelles from the mitochondrial cristae.

The sub-mitochondria particles may be lyophilized.

The mitochondrial enzymes may be selected from NADH-dehydrogenase, coenzyme-Q-cytochrome C reductase, and cytochrome C oxidase.

The electron donor may include at least one selected from the group consisting of NADH and NADPH.

The pH indicator may change color in the pH range of 5.2 to 10.

The pH indicator may be selected from the group consisting of bromocresol purple, bromothymol blue, phenol red, thymol blue, and phenolphthalein.

The pH indicator may be adjusted in the pH range of 7.5 to 10.

The pH indicator may be adjusted in the pH range of 9 to 10.

In the kit of the present invention, sub-mitochondria particles, competent mitochondrial enzymes, an electron donor, toxic materials, and one or more pH indicators are described as in the method of detecting toxic materials according to the present invention.

The present invention will now be described in greater detail with reference to the following examples. The following examples are for illustrative purposes and are not intended to limit the scope of the invention.

### <Example 1>

### Detecting toxicity according to the present invention

Toxic materials are detected using the method according to the present invention. Ethanol was used as a toxic material and sorbitol, which is known as a material having no toxicity to a cell, was used.

NaOH was adequately added to a pH indicator, phenol red, to adjust the pH to 10. Then, 20% of sorbitol, 2.5% of ethanol, and 20% of ethanol were added to each 0.5 mg/ml of SMP (submitochondrial particle)(Havard Bioscience), 6 mM of NADH, and 50 mM of KCl and were allowed to react for 10 minutes at ambient temperature. The sample reaction obtained by the above process and a phenol red solution were mixed together with the weight ratio of 1:1 to identify the color of the mixture solution.

The results are shown in FIG. 4. Referring to FIG. 4, when 20 % of sorbitol (non-toxicity) and 2.5 % of ethanol were added, the color of the mixture solution remained cherry. However, when 20 % of ethanol was added, the color of the mixture solution changed to red orange.

According to this result, phenol red is used to positively detect a high degree of toxicity but a low degree of toxicity cannot be detected efficiently.

### <Predictive Example 1 >

### Detecting toxicity according to the present invention

Toxic materials are detected in the same manner as in Example 1, except that phenolphthalein is used as a pH indicator instead of phenol red.

The results are shown in FIG. 6. Referring to FIG. 6, when 20 % of sorbitol (non-toxicity) is added, the color of the mixture solution remains pink and when 2.5 % of ethanol is added, the color of the mixture solution changes from pink to colorless (result is not illustrated).

According to this result, phenolphthalein is used to positively detect a low degree of toxicity but a toxicity level measurement cannot be derived from the result itself.

### <Predictive Example 2>

### Detecting the degree of toxicity according to the present invention

Toxic materials are detected in the same manner as in Example 1, except that both phenol red and phenolphthalein are used as pH indicators instead of phenol red alone.

The results are shown in FIG. 7. Referring to FIG. 7, when 20 % of sorbitol (non-toxic) is added, the color of two mixture solutions remains the same. When 2.5 % of ethanol is added, the color of the mixture solution in which phenol red is added remains the same but the color of the mixture solution in which phenolphthalein is added changes from pink to colorless. When 20 % of ethanol is added, the color of two mixture solutions in which phenol red and phenolphthalein are added change from cherry and pink to red orange and colorless, respectively.

According to this result, when more than two kinds of pH indicators are used, toxic materials can be positively detected and the degree of toxicity also can be measured.

As described above, all kinds of toxic materials can be detected as well as the presence of toxic materials and their degree of toxicity. In addition, no special equipment or apparatus is required and thus, simplification of the method is possible. When materials desired to be detected exist, the method according to the present invention is the same as a general positive detection mechanism and thus, commercialization is also possible.

While the present invention has been particularly shown and described with reference to exemplary embodiments thereof, it will be understood by those of ordinary skill in the art that various changes in form and details may be made therein without departing from the spirit and scope of the present invention as defined by the following claims.

## Claims

1. A method of positively detecting toxic materials within a sample comprising the steps of:
(i) contacting sub-mitochondria particles having competent mitochondrial enzymes derived from the inner membranes of mitochondria and an electron donor which transmits electrons to an electron transfer system of the sub-mitochondria particles, with a sample to be tested;
(ii) adjusting the pH of one or more pH indicators to a pH higher than the pH of the sample reaction of (i);
(iii) adding the pH indicator(s) of (ii) to the sample reaction of (i); and
(iv) identifying color changes of one or more pH indicators, wherein a color change indicates the presence of a toxic material and non-toxicity is indicated when no color change occurs.

2. The method of claim 1, wherein the sub-mitochondria particles are manufactured by sonicating whole mitochondria to generate micelles from the mitochondrial cristae.

3. The method of claim 1, wherein the mitochondrial enzymes are selected from NADH-dehydrogenase, coenzyme-Q-cytochrome C reductase, and cytochrome C oxidase.

4. The method of claim 1, wherein the electron donor includes at least one selected from the group consisting of NADH and NADPH.

5. The method of claim 1, wherein the pH indicator changes color in the pH range of 5.2 to 10.

6. The method of claim 1, wherein the pH indicator is selected from the group consisting of bromocresol purple, bromothymol blue, phenol red, thymol blue, and phenolphthalein.

7. The method of claim 1, wherein the pH indicator is adjusted in the pH range of 7.5 to 11.

8. The method of claim 1, wherein the pH indicator is adjusted in the pH range of 9 to 11.

9. The method of claim 1, wherein the degree of toxicity is indicated by a change of the color of one or more pH indicators and non-toxicity is indicated when the color of a pH indicator is not changed.

10. The method of claim 1, wherein one or more pH indicators are formed of more than two pH indicators and the degree of toxicity is indicated by the color changes of two pH indicators.

11. A kit for positively detecting toxic materials within a sample comprising:
sub-mitochondria particles having competent mitochondrial enzymes derived from the inner membranes of mitochondria;
an electron donor which transmits electrons to an electron transfer system of the sub-mitochondria particles; and
one or more pH indicators.

12. The kit of claim 11, wherein the sub-mitochondria particles are manufactured by sonicating whole mitochondria to generate micelles from the mitochondrial cristae.

13. The kit of claim 11, wherein the sub-mitochondria particle is lyophilized.

14. The kit of claim 11, wherein the mitochondrial enzyme is selected from NADH-dehydrogenase, coenzyme-Q-cytochrome C reductase, and cytochrome C oxidase.

15. The kit of claim 11, wherein the electron donor includes at least one selected from the group consisting of NADH and NADPH.

16. The kit of claim 11, wherein the pH indicator changes color in the pH range of 5.2 to 10.

17. The kit of claim 11, wherein the pH indicator is selected from the group consisting of bromocresol purple, bromothymol blue, phenol red, thymol blue, and phenolphthalein.

18. The kit of claim 11, wherein the pH indicator is adjusted in the pH range of 7.5 to 10.

19. The kit of claim 11, wherein the pH indicator is adjusted in the pH range of 9 to 10.
